(19) Europäisches Patentamt
European Patent Office
Office européen des brevets

(11) Veröffentlichungsnummer: **0 142 788**
**B1**

(12) # EUROPÄISCHE PATENTSCHRIFT

(45) Veröffentlichungstag der Patentschrift: **24.09.86**

(51) Int. Cl.⁴: **C 07 C 102/00**, C 07 C 103/44

(21) Anmeldenummer: **84113516.3**

(22) Anmeldetag: **09.11.84**

(54) **Verfahren zur Herstellung von reinen 3-Acetylaminoanilinen.**

(30) Priorität: **19.11.83 DE 3341883**

(43) Veröffentlichungstag der Anmeldung: **29.05.85 Patentblatt 85/22**

(45) Bekanntmachung des Hinweises auf die Patenterteilung: **24.09.86 Patentblatt 86/39**

(84) Benannte Vertragsstaaten: **CH DE FR GB LI**

(56) Entgegenhaltungen: **EP-A-0 007 738**
**EP-A-0 011 048**

(73) Patentinhaber: **HOECHST AKTIENGESELLSCHAFT, Postfach 80 03 20, D-6230 Frankfurt am Main 80 (DE)**

(72) Erfinder: **Papenfuhs, Theodor, Dr., Heinrich-Bleicher- Strasse 40, D-6000 Frankfurt am Main 50 (DE)**
Erfinder: **Hintzmann, Manfred, Dr., Sachsenring 48, D-6238 Hofheim am Taunus (DE)**

EP 0 142 788 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Die vorliegende Erfindung betrifft ein Verfahren zur Herstellung reiner 3-Acetylamino-aniline der allgemeinen Formel (1)

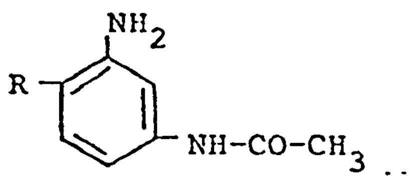

(1)

in welcher R ein Wasserstoffatom oder eine Alkoxygruppe von 1 - 4 Kohlenstoffatomen bedeutet. Verbindungen der vorstehend genannten Formel (1) stellen wertvolle Vorprodukte für eine Vielzahl technisch bedeutender Azodispersionsfarbstoffe, insbesondere für die Gruppe der blauen Handelsfarbstoffe vom Typ des C.I. Disperse Blue 79 dar.

Die technische Herstellung der inredestehenden Verbindungen erfolgt bisher durch Acetylierung von p-Alkoxyanilinen, Nitrierung der dabei gebildeten N-Acetyl-palkoxyaniline in Gegenwart von Lösungsmitteln und abschließende Reduktion der entstandenen 4-Acctylamino-2-nitro-alkoxybenzole bzw., wenn R ein Wasserstoffatom ist, durch partielle Schwefelreduktion von 1,3-Dinitrobenzol, Acetylierung des erhaltenen 3-Nitroanilins und abschließende Hydrierung.

Da diese bekannten Herstellungswege sehr teuer sind, hat es nicht an Versuchen gefehlt, die gesuchten 3-Acetylamino-aniline auf andere, billigere Weise zu erhalten. Als günstigste Synthese für 3-Acetylamino-6-alkoxyaniline wird in der Europäischen Patentschrift 0011048 ein Eintopf-Verfahren vorgeschlagen, bei dem 2,4-Dinitrochlorbenzol mit Alkanolen/ätznatron zu 2,4-Dinitroalkoxy-benzolen umgesetzt, diese zu 2,4-Diaminoalkoxybenzolen reduziert und abschließend mit Acetylierungsmitteln, vorzugsweise Acetanhydrid, möglichst selektiv zu 3-Acetyl-amino-6-alkoxyanilinen umgesetzt werden. Ein analoger Weg (katalytische Reduktion von 1,3-Dinitrobenzol in Alkanolen mit nachfolgender möglichst selektiver Monoacetylierung) bietet sich für 3-Acetylamino-anilin (mit R = H) an. Diese Verfahren sind zwar erheblich wirtschaftlicher, erlauben aber nicht, die auf den erstgenannten technischen Wegen erzielbaren Reinheiten der Zielprodukte der allgemeinen Formel (1) zu erreichen. Die rein selektive Monoacetylierung nur einer Aminogruppe in 1,3-Diaminobenzolen ist nicht möglich. In jedem Fall wird neben noch nicht umgesetztem Diamin das unerwünschte Bisacetylamin mitgebildet. Diese Nebenkomponenten (im günstigsten Fall beim Verfahren der Europäischen Patentschrift 0011C48 in einer Menge von jeweils einigen Prozent) sind im vorgeschlagenen Aufarbeitungsprozeß (Abdestillieren des Alkanols mit nachfolgender Kristallisation aus Wasser) nicht abtrennbar. Während das enthaltene 1,3-Diaminobenzol (-derivat) sich wegen großer thermischer Instabilität bei der Aufarbeitung größtenteils zu schwarzen Folgeprodukten unbekannter Konstitution zersetzt, bleibt das Bis-acetylamin dabei unverändert. Im isolierten 3-Acetylamino-anilin (-derivat) sind sowohl die schwarzen Zersetzungsprodukte des Diamins, als auch die diacetylierten Nebenkomponenten nahezu vollständig enthalten, wodurch auf diesem wirtschaftlich vorteilhaften Weg gegenüber den erstgenannten technischen Verfahren nur qualitativ erheblich schlechtere Zielprodukte zugänglich sind, die bei der Weiterverarbeitung auf Farbstoffe vom Typ des C.I. Disperse-Blue 79 verfahrenstechnische Probleme aufwerfen und das in ihnen enthaltene Bis-acetylamin in unerwünschter Weise an das Abwasser abgeben.

Es bestand daher die Aufgabe, zweckmäßigerweise unter Anwendung eines wirtschaftlich optimalen Eintopf- Verfahrens (z.B. gemäß der Europäischen Patentschrift 0011048) frei von Verunreinigungen isolierbare 3-Acetylamino-aniline technisch zugänglich zu machen. Diese Aufgabe wurde durch die vorliegende Erfindung auf folgende Weise gelöst:

Es wurde gefunden, daß man 3-Acetylanino-aniline der vorstehend genannten allgemeinen Formel (1) rein herstellen kann, indem man das in bekannter Weise durch Reduktion von 1,3-Dinitrobenzol in einem Alkanol von 1 - 4 Kohlenstoffatomen erhaltene 1,3-Diaminobenzol bzw. das in bekannter Weise durch Umsetzung von 2,4-Dinitrochlorbenzol mit Ätznatron in einem Alkanol mit 1 - 4 Kohlenstoffatomen und anschließende Reduktion erhaltene 2,4-Diamino-alkoxybenzol in bekannter Weise in dem genannten Alkanol, vorzugsweise mit Acetanhydrid, zum 1-Amino-3-acetylamino-benzol bzw.zum 2-Amino-4-acetylamino-alkoxy-benzol monoacetyliert, und die in der alkanolischen Lösung, die Wasser enthalten kann, angefallene Verbindung der genannten allgemeinan Formel (1) selektiv und nahezu quantitativ mittels Halogenwasserstoff in Porm des entsprechenden Hydrohalogenids ausfällt und durch Filtration abtrennt.

Hierbei bleiben sämtliche Begleitsubstanzen (Diaminobenzole und/oder ihre Zersetzungsprodukte sowie

Bisacetylamine) vollständig in Lösung und können durch Filtration abgetrennt werden.

Das erfindungsgemäße Verfahren ist insofern äußerst überraschend, als nach bisheriger Kenntnis aromatische Amine, insbesondere durch Amino-, Alkoxy- und/oder Acetylaminogruppen substituierte Aniline, in Wasser und Alkoholen leichtlösliche Hydrohalogenide und, wenn überhaupt schwerlösliche Salze, dann allenfalls schwerlösliche sulfate zu bilden vermögen. So werden bestimmte Toluidine, Chloraniline und Anisidine, z.B. 2,4-Diaminotoluol, -chlorbenzol, -anisol und -phenetol, technisch wichtige Vorprodukte für Haar- und Pelzfarbstoffe, generell aus alkoholischer Lösung als schwerlösliche Sulfate isoliert, da die entsprechenden Hydrohalogenide leichtlöslich sind (vgl. z.B. Monatshefte für Chemie 22, 119; Recueil des trav. chim. des Pays Bas 47, 185).

3-Acetylamino-6-alkoxyaniline, bei denen der Alkoxyrest mehr als 1 0-Atom (wie z.B. der β-Methoxyethoxyrest) und/oder die Acylaminogruppe mehr als 2-C-Atome (wie z.B. die Propionyl- oder Butyroylaminogruppe) enthält, sowie 6-Acylamino-4-amino-1,3-xylole bilden dagegen in Alkoholen weder schwerlösliche Sulfate noch schwerlösliche Hydrohalogenide.

Es war daher weder voraussehbar, noch nach heutigem Kenntnisstand anzunehmen, daß 3-Acetylamino-aniline der allgemeinen Formel (1) in gegebenenfalls Wasserhaltigen niedermolekulären Alkanolen äußerst schwerlösliche Hydrohalogenide bilden und als solche von allen Verunreinigungen, die dazu nicht in der Lage sind (Diaminobenzole, Bisacetylamine), durch Filtration abgetrennt werden können.

Dies gilt umso mehr, als für eine der erfindungsgemäß fällbaren Verbindungen, nämlich das 3-Acetylaminoanilinhydrochlorid, sogar eine gute Löslichkeit in Methanol in der Literatur behauptet wird (JACS 39, 1948).

Als erfindungsgemäß verwendbare Alkanole seien die zumindest teilweise mit Wasser mischbaren niederen Alkanole, wie Methanol, Ethanol, n- und Isopropanol sowie die isomeren Butanole genannt. Bei den erfindungsgemäß fällbaren Alkoxyderivaten sind naturgemäß diejenigen Alkanole von besonderem Vorteil, die zum Chloraustausch in dem in der Europäischen Patentschrift 0011048 beschriebenen Eintopfverfahren im Überschuß eingesetzt werden und in den Folgestufen (Reduktion und Acetylierung) als Lösungsmittel dienen, da in diesen Fällen der Eintopfreaktion direkt und ohne Zwischenisolierung der rohen 3-Acetylamino-6-alkoxy-aniline eine Isolierung der gesuchten, von Nebenprodukten befreiten Hydrohalogenide der Zielprodukte angeschlossen werden kann.

Da die Schwerlöslichkeit der erfindungsgemäß fällbaren Hydrohalogenide auch in wasserhaltigen Alkanolen gegeben ist, kann die Salzbildung sowohl durch Einleiten von gasförmigem Halogenwasserstoff, wie beispielsweise Bromwasserstoff oder vorzugsweise Chlorwasserstoff, in der mindestens stöchiometrischen Menge in die gegebenenfalls Wasser enthaltende alkoholische Lösung der Verbindung der allgemeinen Formel (1), als auch, besonders vorteilhaft, durch Versetzen dieser Lösung mit der mindestens stöchiometrischen Menge wäßriger Halogenwasserstoffsäure, beispielsweise wässriger Bromwasserstoffsäure oder vorzugsweise Salzsäure, erfolgen.

Erfindungsgemäß sind Wassergehalte der als Fällmedium vorliegenden Alkanole bis 30 Gew.-%, bei mit Wasser nur begrenzt mischbaren Alkanolen bis zur Sättigungsgrenze ohne Ausbeuteminderung tolerierbar.

Als Fällmittel sind gasförmige Halogenwasserstoffe oder ihre wäßrigen Lösungen bis zu Konzentrationen, die die obengenannten Wassergehalte des Fällmediums einzuhalten gestatten, geeignet. Im Prinzip können alle Halogenwasserstoffe eingesetzt werden. Aus Wirtschaftlichkeitsgründen sind Chlor- und Bromwasserstoff, insbesondere Chlorwasserstoff, bevorzugt.

Die Fällung der Hydrohalogenide der 3-Acetylamino-aniline der allgemeinen Formel (1) kann in weiten Temperaturbereichen erfolgen. Als obere Grenze gilt der Siedepunkt des Fällmediums. Bevorzugt sind Temperaturen von 20 bis 50°C, da bei höherer Temperatur und langer Verweilzeit eine teilweise Verseifung der Acetylaminogruppe nicht immer ausgeschlossen werden kann.

Die Isolierung der gefällten 3-Acetylamino-anilin-hydro-halogenide erfolgt zweckmäßig bei niederen Temperaturen, beispielsweise bei 0 bis 20°C, da bei erhöhten Temperaturen erwartungsgemäß die Löslichkeit der Hydrohalogenide, wenn auch in überraschend geringem Umfang, zunimmt und dadurch bei höheren Isoliertemperaturen Ausbeuteverluste in Kauf genommen werden müssen.

Der zur Fällung eingesetzte Halogenwasserstoff wird in mindest stöchiometrischer Menge verwandt. Überschüsse schaden nicht, da die Hydrohalogenide der Begleitsubstanzen im Fällmedium leichtlöslich sind, belasten aber unter Umständen die Wirtschaftlichkeit des Reinigungsverfahrens.

Eine nahezu alleinige Fällung des 3-.Acetylamino-anilin-hydrohalogenids der genannten allgemeinen Formel (1) gelingt auch, wenn in der Fäll-Lösung, wie beim Verfahren der Europäischen Patentschrift 0C11C48 bevorzugt, Alkalioder Erdalkalimetallsalze, z.B. Magnesiumacetat, enthalten sind. Dies insbesondere dann, wenn zur Fällung wässrige Halogenwasserstoffsäuren eingesetzt werden, da dann ein wässrig alkoholisches Fällmedium resultiert, in dem die gegebenenfalls teilweise gebildeten Alkali- oder Erdalkalimetallhalogenide größtenteils gelöst bleiben. In geringem Umfang ungelöste, und damit mit den Zielprodukten isolierte Salze stören bei Folgereaktionen nicht und sind daher tolerierbar, da sie im Zuge der Farbstoffsynthese problemlos abgetrennt werden können.

Das erfindungsgemäße Verfahren ist als neu und überraschend zu erachten. Dies umso mehr, als die Zielprodukte in Alkanolen in keinem Fall schwerlösliche Sulfate zu bilden vermögen und sich bereits darin von verwandten vorgenannten Anilinderivaten unterscheiden. Es lag weder nahe, noch war es voraussehbar, daß bei dieser Sachlage die sonst generell erheblich leichter löslichen Hydrohalogenide zu praktisch quantitativer Fällung der in Alkanolen gelösten 3-Acetylamino-aniline geeignet sind und durch Filtration von in Lösung verbleibenden Nebenkomponenten abgetrennt werden können.

Das erfindungsgemäße Verfahren erlaubt auf wirtschaftlich optimalem Wege, ausgehend von 2,4-Dinitrochlorbenzol bzw. 1 3-Dinitrobenzol, in einer Eintopfreaktion die technisch wichtigen 3-Acetylamino-aniline der allgemeinen Formel (1) in Form ihrer Hydrohalogenide in einer Reinheit herzustellen, wie sie bisher nur auf den bedeutend aufwendigeren Wegen über die Nitrierung von 4-Acetylamino-alkoxy-benzolen bzw. über die Acetylierung von 3-Nitranilin erzielbar waren. Zusätzlich fallen die abgetrennten Verunreinigungen nach Regeneration der Alkohole als konzentrierter Destillationssumpf an, der vorteilhaft durch Verbrennung entsorgt werden kann, was eine bedeutende Abwasserentlastung zur Folge hat. Damit stellt das erfindungsgemäße Verfahren einen erheblichen technischen Fortschritt gegenüber dem Stand der Technik dar.

Es ist natürlich auch möglich, die Verbindungen der allgemeinen Formel (1) vor ihrer erfindungsgemäßen Fällung und Abfiltration aus der gegebenenfalls alkanolischen Lösung auf beliebigem anderen Weg herzustellen, mit angefallenen Verunreinigungen zu isolieren und die zwischenisolierten Produkte in einem Alkanol von 1 - 4 Kohlenstoffatomen zu lösen und dann erfindungsgemäß in Form der Hydrohalogenide zu isolieren. Es ist jedoch zweckmäßig und auch vorteilhaft, die Ausgangsverbindungen 1,3-Dinitrobenzol bzw. 2,4-Dinitrochlorbenzol in der angegebenen, an sich bekannten Weise im Zuge der wirtschaftlich optimalen Eintopfreaktion in die Verbindungen der genannten Formel (1) überzuführen, wobei diese aufgrund des Einsatzes von Alkanolen mit 1 - 4 Kohlenstoffatomen in den vorausgehenden Reaktionsstufen bereits in den gegebenenfalls wasserhaltigen Alkanolen gelöst anfallen und daraus erfindungsgemäß isoliert werden können.

Die so zugänglichen reinen 3-Acetylamino-anilin-hydro-halogenide können meist als solche für Folgereaktionen eingesetzt werden. Wenn erforderlich, bereitet es keine Schwierigkeiten, aus ihnen durch Neutralisation mittels Alkalien, z.B. in wäßriger Lösung oder Suspension, die freien reinen 3-Acetylamino-aniline herzustellen und diese gegebenenfalls durch Filtration von den resultierenden wäßrigen Alkalimetallhalogenidlösungen abzutrennen.

Die nachfolgenden Beispiele sollen die Erfindung näher erläutern, ohne sie auf die darin beschriebenen Ausführungen einzuschränken:

**Beispiel 1**

Eine gemäß Beispiel 1 der Europäischen Patentschrift 0011048 durch Umsetzung von 213 Teilen 2,4-Dinitrochlorbenzol mit 42 Teilen Ätznatron in 632 Teilen Methanol, anschließende Hydrierung an Pd/Kohle bei 60°C und nachfolgende Acetylierung mit 98 Teilen Essigsäureanhydrid in Gegenwart von 22 Teilen Magnesiumoxid bei 0 - 5°C erhaltene dunkle Lösung von 6-Methoxy-3-acetylamino-anilin, die nahh der "High performance liquid chromatography analysis" ("HPLC-Analyse") neben 153 Teilen Zielprodukt 8,9 Teile 2,4-Bisacetylaminoanisol und 3,5 Teile 2,4-Diaminoanisol, sowie schwarze oxidativ gebildete Zersetzungsprodukte enthält, wird bei 20 - 25°C unter Rühren innerhalb von 30 Minuten tropfenweise mit 197 Teilen 37%iger Salzsäure versetzt. Man rührt 1 Stunde nach, kühlt auf 0 - 5°C ab und isoliert das ausgefallene 6-Methoxy-3-acetylamino-anilin-hydrochlorid durch Filtration. Man wäscht zweimal mit 100 Teilen Methanol und erhält nach dem Trocknen im Vakuum 188,5 Teile 6-Methoxy-3-acetylamino-anilin-hydrochlorid, das neben ca. 5 % Magnesiumchlorid keine durch Dünnschichtchromatographie ("DC") oder HPLC-Analyse nachweisbaren organischen Nebenprodukte enthält. Das isolierte Produkt entspricht somit 179,1 Teilen 100 %igem 6-Methoxy-2-acetyl-amino-anilin-hydrochlorid, was einer Ausbeute von 82,7 % d.Th., bezogen auf eingesetztes 2,4-Dinitrochlorbenzol, entspricht.

Durch zweimaliges Waschen des methanolfeuchten Filterkuchens mit je 100 Teilen Eiswasser und nachfolgendes Trocknen im Vakuum erhält man ein salzfreies Reinprodukt (Gehalt an 6-Methoxy-3-acetylamino-anilin durch HPLC-Analyse 99 %) in einer Ausbeute von 80,2 % d.Th. (173,6 Teile).

**Beispiel 2**

194 Teile gemäß Beispiel 3 der Europäischen Patentschrift 0011048 erhaltenes getrocknetes rohes 6-Ethoxy-3-acetylamino-anilin (neben 175,5 Teilen Zielprodukt 9,5 Teile 2,4-Bis-acetylaminophenetol und 3,5 Teile 2,4-Diaminophenetol neben dunklen, oxidativ gebildeten Verunreinigungen enthaltend), werdem in 250 Teilen Ethanol von 50°C unter Rühren gelöst. Nach Abkühlen auf 20 - 30°C gast man innerhalb 1 Stunde ca. 35 - 40 Teile Chlorwasserstoffgas in die dunkle Lösung, rührt 1 Stunde nach und isoliert das ausgefallene 6-Ethoxy-3-acetylamino-anilin-hydrochlorid nach Abkühlen auf 0 - 5°C durch Filtration auf einer Nutsche. Man wäscht zweimal mit je 75 Teilen Ethanol, trocknet im Vakuum und erhält 204,0 Teile 6-Ethoxy-3-acetylamino-anilin-hydrochlorid (durch HPLC-Analyse keine Verunreinigungen nachweisbar, Reingehalt durch Diazotierung 99,2%), was einer Ausbeute von 88,5 % d.Th. (bezogen auf eingesetztes Rohprodukt) bzw. von 97,8 % d.Th. (bezogen auf darin enthaltenes Zielprodukt) entspricht.

Verwendet man anstelle von 250 Teilen Ethanol 150 Teile Methanol und verfährt im übrigen wie vorstehend beschrieben, so erhält man 202,8 Teile 6-Ethoxy-3-acetylamino-anilin-hydrochlorid von gleicher Qualität (Ausbeute 88,0 % d.Th. auf Rohprodukt bzw. 97,2 % d.Th. auf darin enthaltenes Zielprodukt).

**Beispiel 3**

Eine Lösung von 168 Teilen 1,3-Dinitrobenzol in 400 Teilen Methanol wird zusammen mit 4 Teilen 5 %igem Pd/Kohle-Kontakt in einen 1 1-Autoklav überführt. Nach Spülen mit Stickstoff und nachfolgend Wasserstoff wird bei 40-65°C/20 bar Wasserstoff hydriert. Wenn keine Wasserstoffaufnahme mehr erfolgt, wird auf Raumtemperatur abgekühlt, entspannt und vom Katalysator geklärt.

Die erhaltene wäßrig-methanolische Lösung des m-Phenylendiamins wird mit 22 Teilen Magnesiumoxid versetzt, auf 0 - 5°C abgekühlt und dann innerhalb von 2 Stunden tropfenweise mit 100 Teilen Essigsäureanhydrid acetyliert. Es resultiert eine dunkle Lösung, die nach HPLC-Analyse neben 133,5 Teilen 3-Acetylamino-anilin 9,4 Teile 1,3-Bisacetylaminobenzol, 1,9 Teile m-Phenylendiamin und schwarze, durch oxidative Zersetzung gebildete Verunreinigungen enthält.

Zu ihr tropft man bei 10 - 15°C innerhalb 30 Minuten 150 Teile 30 %ige Salzsäure, rührt 30 Minuten nach, kühlt auf 0 bis 5°C und filtriert das ausgefallene 3-Acetylamino-anilin-hydrochlorid ab. Der Filterkuchen wird zweimal mit je 75 Teilen Methanol gewaschen und im Vakuum getrocknet. Man erhält 165 Teile Feststoff, der neben ca. 4 % Magnesiumsalzen keine durch DC oder HPLC-Analyse nachweisbaren organischen Nebenprodukte enthält und durch Diazotierung einen Reingehalt von 96,0 % (≙ 158,4 Teile 3-Acetylamino-anilin-hydrochlorid) aufweist, was einer Ausbeute von 84,9 % d.Th., bezogen auf 1,3-Dinitrobenzol, entspricht.

Durch zweimaliges Waschen des methanolfeuchten Filterkuchens mit je 60 Teilen Eiswasser erhält man ein salzfreies Produkt mit einem Diazotierwert von 99,4 % (155,2 Teile entsprechend 83,2 % d.Th.).

Verwendet man anstelle der 400 Teile Methanol 500 Teile Isopropanol und arbeitet im übrigen wie vorstehend beschrieben, so erhält man das 3-Acetylamino-anilin-hydrochlorid in vergleichbarer Ausbeute und Qualität.

**Beispiel 4**

208 Teile nach Beispiel 7 der Europäischen Patentschrift 0011048 erhaltenes getrocknetes rohes 6-n-Propoxy-3-acetylamino-anilin (neben 190,3 Teilen Zielprodukt 8,8 Teile 2,4-Bisacetylamino-1-n-propoxybenzol und 3,5 Teile 2,4-Diamino-1-n-propoxybenzol sowie geringe Mengen Magnesiumacetat und oxidative Zersetzungsprodukte enthaltend), werden in 300 Teilen Ethanol unter Rühren bei 50°C gelöst und nach Abkühlen auf 25°C innerhalb 1,5 Stunden tropfenweise mit 170 Teilen 48 %iger Bromwasserstoffsäure versetzt. Der ausgefallene Niederschlag wird bei 0 - 5°C abgesaugt, mit zweimal 60 Teilen Ethanol gewaschen und im Vakuum getrocknet. Man erhält 257,1 Teile 6-n-Propoxy-3-acetylamimo-anilin-hydrobromid (durch HPLC keine Verunreinigungen nachweisbar, Reingehalt durch Diazotierung 99,4 %), was einer Ausbeute von 88,9 % d.Th. (bezogen auf rohes Einsatzprodukt), bzw. von 97,2 % d.Th. (bezogen auf darin enthaltenes Zielprodukt) entspricht.

Verwendet man anstelle von 300 Teilen Ethanol 350 Teile Isobutanol und arbeitet im übrigen wie vorstehend beschrieben, so erhält man das 6-n-Propoxy-3-acetylamino-anilin-hydrobromid in vergleichbarer Qualität bei ca. 2 % niedrigerer Ausbeute.

**Beispiel 5**

180 Teile eines schwarzen, rohen 6-Methoxy-3-acetylamino-anilins (hergestellt auf dem Verfahrensweg der Europäischen Patentschrift 0011048, aber unter Zusatz von 10 % Wasser vor der Hydrierung und unter Steigerung der Acetylierungstemperatur auf 20 - 25°C, wodurch eine weniger glatt verlaufende Reduktion der Nitrogruppen und deutlich geringere Selektivität in der Monoacetylierung resultierte), das nach HPLC neben 124 Teilen Zielprodukt und 22 Teilen 2,4 Bisacetylamino-anisol erhebliche Mengen (ca. 30 - 35 Teile) nicht identifizierbare Neben- und oxidativ gebildete Zersetzungsprodukte enthielt, wurden in 500 Teilen Methanol bei 50°C gelöst, nach Abkühlen auf 20 - 25°C in 3C Minuten unter Rühren tropfenweise mit 100 Teilen 30 %iger Salzsäure versetzt und dann 1,5 Stunden nachgerührt. Das nach Abkühlen auf 0 - 5°C durch Filtration auf einer Nutsche isolierte Produkt wurde zweimal mit je 100 Teilen Methanol gewaschen und im Vakuum getrocknet. Man erhält 144 Teile nahezu farbloses 6-Methoxy-3-acetylamino-anilin-hydrochlorid, das nach HPLC-Analyse keinerlei organische Verunreinigungen enthielt und dessen Reingehalt durch Diazotierung 99,3 % betrug, was einer Ausbeute von 96,8 % d.Th., bezogen auf im Rohprodukt enthaltenes 6-Methoxy-3-acetyl-amino-anilin entspricht.

Durch einstündiges Verrühren des methanolischen Filterkuchens mit Kaltwasser, Zutropfen von 33 %iger Natronlauge bis pH 7,5 und Abkühlen der erhaltenen Suspension auf 0-5°C kann nach Filtration, zweimaligem Waschen mit je 50 Teilen Eiswasser und Trocknen im Vakuum das freie 6-Methoxy-3-amino-anilin in einer Ausbeute von 92,8 % d.Th., bezogen auf im Rohprodukt enthaltenes Zielprodukt, erhalten werden.

**Beispiel 6**

Die gemäß Europäischer Patentschrift 0011048 durch Umsetzung von 213 Teilen 2,4-Dinitrochlorbenzol mit 632 Teilen n-Butanol und 42 Teilen Ätznatron mach Hydrierung und Acetylierung erhaltene dunkle butanolische Lösung vom 6-n-Butoxy-3-acetamino-anilin, welche nach HPLC-Analyse neben 195 Teilen Zielprodukt 11,9 Teile 2,4-Bis-acetyl-amino-1-n-butoxy-benzol umd 8,1 Teile 2,4-Diamino-1-n-butoxybenzol, sowie unbekannte, oxidativ entstandene Zersetzungsprodukte enthält, wird durch 1-stündiges Eingasen von 35 - 40 Teilen Chlorwasserstoff bei 20 - 30°C gefällt, der Niederschlag nach Abkühlen auf 0 - 5°C abgesaugt, zweimal mit je 50 Teilen Methanol gewaschen und im Vakuum getrocknet. Man erhält 221,2 Teile 6-n-Butoxy-3-acetylamino-anilin-hydrochlorid, das neben ca. 3 % Magnesiumchlorid keinerlei durch HPLC-Analyse nachweisbare Verunreinigungen enthält (Reingehalt durch Diazotierung: 95,8 %). Dies entspricht einer Ausbeute vom 214,1 Teilen 6-n-Butoxy-3-acetylamino-amilin-hydrochlorid 100 %ig (82,8 % d.Th., bezogen auf 2,4-Dinitrochlorbenzol).

**Beispiel 7**

(Vergleichsbeispiel)

Versetzt man eine gemäß Beispiel 4 der Europäischen Patentschrift 0011048 erhaltene methanolische Lösung von 6-Methoxy-3-propionylamino-anilin mit 30 %iger Salzsäure, so tritt keinerlei Fällung auf. Allfällige Nebenprodukte sind auf diesem Wege nicht abtrennbar.

Das gleiche negative Ergebnis wird erhalten, wenn eine alkoholische Lösung von 6-B-Methoxyethoxy-3-acetaminoanilin (Verbindung 8 aus der Europäischen Patentschrift 0011048) oder von 6-β-Hydroxyethoxy-3-acetamino-anilin (Verbindung 15 aus der Europäischen Patentschrift 0011048) mit gasförmigem oder in Wasser gelöstem Chlorwasserstoff versetzt wird.

**Patentansprüche**

1. Verfahren zur Herstellung reiner 3-Acetylamino-aniline der allgemeinen Formel (1)

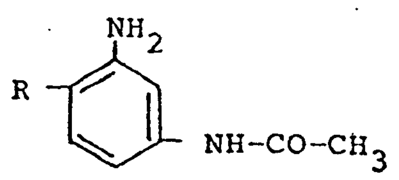

(1)

in welcher R ein Wasserstoffatom oder eine Alkoxygruppe von 1 - 4 Kohlenstoffatomen bedeutet, durch Reduktion von 1,3-Dinitrobenzol in einem Alkanol von 1 - 4 Kohlenstoffatomen zum 1,3-Diaminobenzol bzw. durch Umsetzung von 2,4-Dinitrochlorbemzol mit Ätznatron in einem Alkanol mit 1 - 4 Kohlenstoffatomen und anschließende Reduktion zum 2,4-Diaminoalkoxybenzol, und jeweils anschließende Monoacetylierung in dem genannten Alkanol zum 1-Amino-3-acetylamino-benzol bzw. zum 2-Amino-4-acetylamino-alkoxy-benzol und Isolierung der in der gegebenenfalls Wasser enthaltenden alkanolischen Lösung angefallenen Verbindung der genannten Formel (1), dadurch gekennzeichnet, daß man die Isolierung durch Ausfällen der Verbindung der genannten Formel (1) aus der alkanolischen Lösung mittels Halogenwasserstoff oder wäßriger Halogenwasserstoffsäure in Form des Hydrohalogenids und anschließende Filtration vornimmt.

2. Verfahren nach Anspruch 1, dadurch gekennzeichnet, daß man die Ausfällung der Verbindung der allgemeinen Formel (1) durch Einleiten von gasförmigem Halogenwasserstoff, vorzugsweise Chlorwasserstoff, in der mindestens stöchiometrischen Menge in die gegebenenfalls Wasser enthaltende alkanolische Lösung vornimmt.

3. Verfahren nach Anspruch 1, dadurch gekennzeichnet, daß man die Ausfällung der Verbindung der allgemeinen Formel (1) durch Versetzen der gegebenenfalls Wasser enthaltenden alkanolischen Lösung mit der mindestens stöchiometrischen Menge wässriger Halogenwasserstoffsäure, vorzugsweise Salzsäure, vornimmt.

4. Verfahren nach Ansprüchen 1 - 3, dadurch gekennzeichnet, daß der Wasser gehalt der als Lösungsmittel und Fällmedium dienenden Alkanole bis 30 Gew.-% ausmacht.

5. Verfahren nach Ansprüchen 1 - 3, dadurch gekennzeichnet, daß der Wassergehalt der als Lösemittel und Fällmedium dienenden Alkanole, die mit Wasser nur begrenzt mischbar sind, bis zur Sättigungsgrenze geht.

6. Verfahren nach Anspruch 1, dadurch gekennzeichnet, daß die Ausfällung der Verbindungen der Formel (1) in Form der Hydrohalogenide bei einer Temperatur erfolgt, die nicht höher als der Siedepunkt des Fällmediums ist.

7. Verfahren nach Anspruch 1, dadurch gekennzeichnet, daß man die Ausfällung der Verbindungen der Formel (1) in Form der Hydrohalogenide bei Temperaturen von 20-50° C vornimmt.

**Claims**

1. A process for the preparation of pure 3-acetylamino-anilines of the general formula (1)

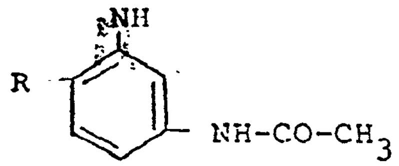

(1)

in which R denotes a hydrogen atom or an alkoxy group with 1 - 4 carbon atoms, by reduction of 1,3-dinitrobenzene in an alkanol with 1 - 4 carbon atoms to 1,3-diaminocenzene or by reaction of 2,4-dinitrochlorobenzene with sodium hydroxide in an alkanol with 1 - 4 carbon atoms and subsequent reduction to the 2,4-diaminoalkoxybenzene, and in each case subsequent monoacetylation in the alkanol mentioned to give the 1-amino-3-acetylamino-benzene or the 2-amino-4-acetylamino-alkoxy-benzene and isolation of the compound of the above formula (1) obtained in the alkanolic solution, which optionally contains water, which comprises carrying out the isolation by precipitation of the compound of the above formula (1), in the form of the hydrohalide, from the alkanolic solution by means of hydrogen halide or aqueous hydrogen halide acid and subsequent filtration.

2. The process as claimed in claim 7, wherein the precipitation of the compound of the general formula (1) is carried out by passing at least the stoichiometric amount of gaseous hydrogen halide, preferably hydrogen chloride, into the alkanolic solution, which optionally contains water.

3. The process as claimed in claim 1, wherein the precipitation of the compound of the general formula (1) is carried out by adding at least the stoichiometric amount of aqueous hydrogen halide acid, preferably hydrochloric acid, to the alkanolic solution, which optionally contains water.

4. The process as claimed in any one of claims 1 to 3, wherein the water content of the alkanols used as the solvent and precipitating medium is up to 30% by weight.

5. The process as claimed in any one of claims 1 to 3, wherein the water content of the alkanols which are used as the solvent und precipitating medium and have only a limited water-miscibility is up to the saturation limit.

6. The process as claimed in claim 1, wherein the precipitation of the compound of the formula (1) in the form of the hydrohalides is carried out at a temperature which is no higher than the boiling point of the precipitating medium.

7. The process as claimed in claim 1, wherein the precipitation of the compound of the formula (1) in the form of the hydrohalides is carried out at a temperature of 20 - 50° C.

**Revendications**

1. Procédé de préparation d'acétylamino-3 anilines pures répondant à la formule générale 1:

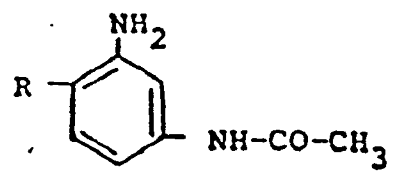

(1)

dans laquelle R représente un atome d'hydrogène ou un radical alcoxy contenant de 1 à 4 atomes de carbone, par réduction du dinitro-1,3 benzène dans un alcanol en $C_1$-$C_4$ conduisant au diamino-1,3 benzène, ou par réaction du dinitro-2,4 chlorobenzène avec l'hydroxyde de sodium dans un alcanol en $C_1$-$C_4$, puis réduction conduisant au diamino-2,4 alcoxybenzène, et ensuite, dans chacun des cas, monoacétylation dans l'alcanol mentionné, conduisant respectivement à l'amino-1 acétylamino-3 benzène ou à l'amino-2 acétylamino-4 alcoxybenzène, et isolement du composé de formule 1 (voir ci-dessus) qui s'est formé dans la solution alcanolique contenant éventuellement de l'eau, procédé caractérisé en ce qu'on effectue l'isolement en faisant précipiter le composé de formule 1 dans la solution alcanolique, au moyen d'un halogénure d'hydrogène ou d'un acide halogénhydrique aqueux, sous la forme de son halogénhydrate, puis en filtrant.

2. Procédé selon la revendication 1 caractérisé en ce qu'on effectue la précipitation du composé de formule générale 1 en introduisant un halogénure d'hydrogène gazeux, de préférence du chlorure d'hydrogène,en une quantité au moins égale à la quantité stoechiométrique, dans la solution alcanolique contenant éventuellement de l'eau.

3. Procédé selon la revendication 1 caractérisé en ce qu'on effectue la précipitation du composé de formule générale 1 en ajoutant, à la solution alcanolique contenant éventuellement de l'eau, une quantité d'acide halogénhydrique aqueux, de préférence d'acide chlorhydrique, au moins égale à la quantité stoechiométrique.

4. Procédé selon l'une quelconque des revendications 1 à 3 caractérisé en ce que la teneur en eau des alcanols servant de solvants et de milieux de précipitation peut aller jusqu'à 30% en poids.

5. Procédé selon l'une quelconque des revendications 1 à 3 caractérisé en ce que la teneur en eau des alcanols qui servent de solvants et de milieux de précipitation et qui ne sont que partiellement miscibles à l'eau peut aller jusqu'à la limite de saturation.

6. Procédé selon la revendication 1 caractérisé en ce que la précipitation des composés de formule 1 sous la forme de leurs halogénhydrates est effectuée à une température qui n'est pas supérieure au point d'ébullition du milieu de précipitation.

7. Procédé selon la revendication 1 caractérisé en ce que la précipitation des composés de formule 1 sous la forme de leurs halogénhydrates est effectuée à des températures de 20 à 50°C.